Europäisches Patentamt

European Patent Office

Office européen des brevets

(11) Publication number: **0 318 498 B1**

# EUROPEAN PATENT SPECIFICATION

(45) Date of publication of patent specification: **03.03.93** (51) Int. Cl.⁵: **C12Q 1/70**, C12N 7/02

(21) Application number: **87905357.7**

(22) Date of filing: **29.07.87**

(86) International application number:
**PCT/US87/01815**

(87) International publication number:
**WO 88/00980 (11.02.88 88/04)**

(54) **HUMAN B LYMPHOTROPIC VIRUS (HBLV) ISOLATION AND PRODUCTS.**

(30) Priority: **04.08.86 US 892423**
**29.08.86 US 901602**

(43) Date of publication of application:
**07.06.89 Bulletin 89/23**

(45) Publication of the grant of the patent:
**03.03.93 Bulletin 93/09**

(84) Designated Contracting States:
**AT BE CH DE FR GB IT LI LU NL SE**

(56) References cited:
**WO-A-84/04327**
**WO-A-86/02930**
**WO-A-89/00599**

**SCIENCE, vol. 234, October 31, 1986;**
**S.Z.SALAHUDDIN et al.: "Isolation of a new**
**virus, HBLV, in patients with lym-**
**phoproliferative disorders" pp. 596-601**

**MICROBIOLOGY, 3rd ed., 1980, Harper & Row**
**(Publishers), Harperstown; DAVIS et al.:**
**"Herpesviruses" chapter 55, pp. 1061-1076**

(73) Proprietor: **THE UNITED STATES OF AMERICA**
**as represented by the Secretary, United**
**States Department of Commerce**
**National Technical Information Service, Of-**
**fice of Government Inventions and Patents,**
**5285 Port Royal Road**
**Springfield, Virginia 22161(US)**

(72) Inventor: **SALAHUDDIN, Syed Zaki**
**12600 Newgate Road**
**Potomac, MD 20854(US)**
Inventor: **GALLO, Robert C.**
**8513 Thornden Terrace**
**Bethesda, MD 20817(US)**

(74) Representative: **Lamb, John Baxter et al**
**MARKS & CLERK 57-60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.5x/3.0.1)

## Description

BACKGROUND OF THE INVENTION

A new Human B Lymphotropic Virus (HBLV) was isolated from the peripheral blood leukocytes of six individuals: one HTLV-III seropositive patient with AIDS-related syndrome; one HTLV-III seropositive patient with angio-immunoblastic lymphoadenopathy; one patient with dermatopathic lymphoadenopathy; a patient with mycosis fungoides; a patient with immunoblastic lymphoma; and one patient with acute lymphoblastic leukemia. All six isolates are closely related by antigenic and molecular analysis, and sera from all six virus positive patients reacted immunologically with each virus isolate. See Table 1. In contrast, only four sera from more than 200 randomly selected healthy donors were seropositive. HBLV contains a large double-stranded DNA genome, and is morphologically similar to some members of the Herpes virus group. A detailed morphological analysis of HBLV is given below.

It selectively infects freshly isolated human B-cells, where it induces the appearance of characteristic large, refractile mononucleated or binucleated cells containing nuclear and cytoplasmic inclusion bodies. However, HBLV is distinguishable from all the known human and sub-human primate Herpesviruses by host range, biological effect on infected cells, and by lack of antigenic or genomic relatedness.

Despite morphological similarities, the host range of HBLV contrasted with that of all members of the human Herpesvirus group. For example, attempts to transmit the virus to a number of T and B lymphoblastoid cells lines and to a variety of other cell types, were unsuccessful. In contract, Epstein-Barr Virus (EBV) infects most B-cells and some epithelial cells. Furthermore, other Herpesviruses [e.g., Cytomegalovirus (CMV), Herpes Simplex I and II (HSV) and Variocella-zoster Virus (VZV)] infect a variety of cell types, often inducing cytopathic effects. Other biological comparisons with EBV further emphasized these differences. For example, no EBV nuclear antigens were detected in HBLV-infected cord blood mononuclear cells. This agent has been tentatively named Human B Lymphotropic Virus (HBLV) because of its apparent uniqueness, its isolation from human tissues, and its preference for B-lymphocytes.

TABLE 1

Isolation of HBLV from Peripheral Blood Lymphocytes of Patients with Lymphoma and Lymphadenopathy

| Patient | Description | Serology* | | HBLV Isolation** |
|---|---|---|---|---|
| | | HTLV | HBLV | |
| 1 | RC 29 WM AIDS KS (Kaposi's sarcoma) B cell lymphoma | -III | 1:80 | + |
| 2 | HA 57 WM OHS (open heart surgery) AILD (dermatopathic lymphadenopathy) | - | 1:40 | + |
| 3 | PD 40 WM Dermatopathic Lymphohadenopathy (IV drug abusers) T8 skin infiltrate | -II and -III | 1:80 | + |
| 4 | GS 17 BM T-cell (acute lymphocytic leukemia ALL (T-4) | - | 1:160 | + |
| 5 | RW 66 BM Mycosis Fungoides (T-4) Cutaneous T-cell Lymphoma | - | 1:80 | + |
| 6 | BD 35 BF Immunoblastic Lymphoma | - | 1:80 | - |

\* Serology was done by directed immunofluorescence using as standard a reference virus isolated from patient GS.

\*\* PBL (Peripheral blood leucocytes) from patients were cultured as the primary source of virus. Virus particles were transmitted to fresh human cord blood. Positive cultures were identified by morphology, IF, and EM.

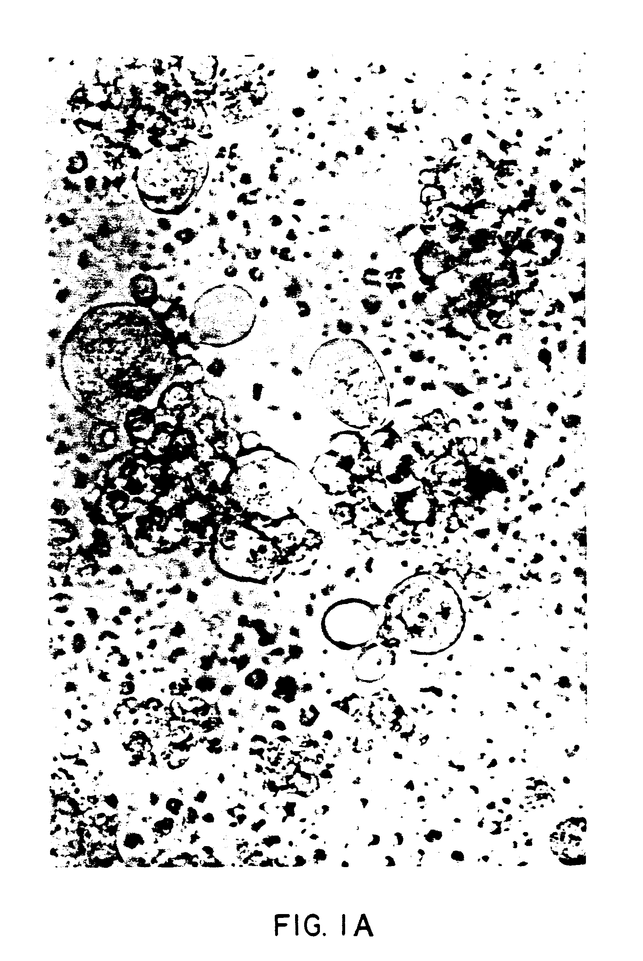

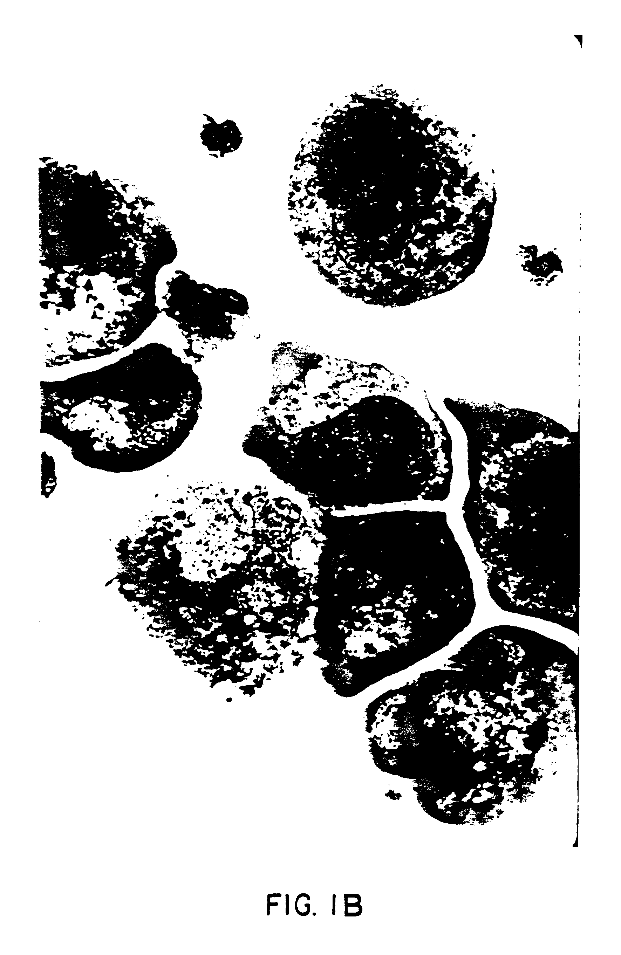

The present invention is the isolation of a new virus, Human B Lymphotropic DNA Virus (HBLV), associated with some malignancies in AIDS and non-AIDS patients. In order to identify and isolate HBLV, fresh peripheral blood mononuclear cells from AIDS patients with associated lymphoproliferative disorders were established in cell culture as described in Figure 1. In the cultures of eight patients, primary cell cultures contained a small number of large, refractile mononucleated or binucleated cells which survive for short periods of time. These cells frequently contained intranuclear and/or intracytoplasmic inclusion bodies.

3

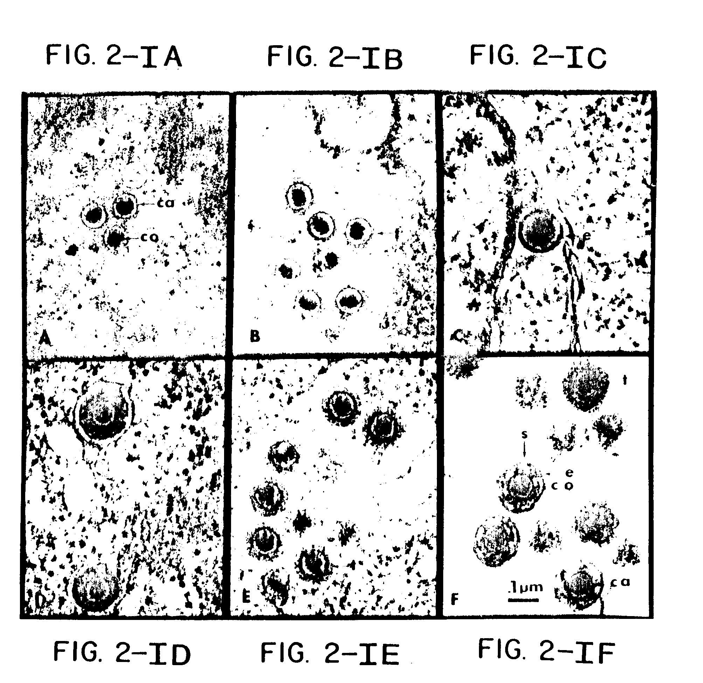

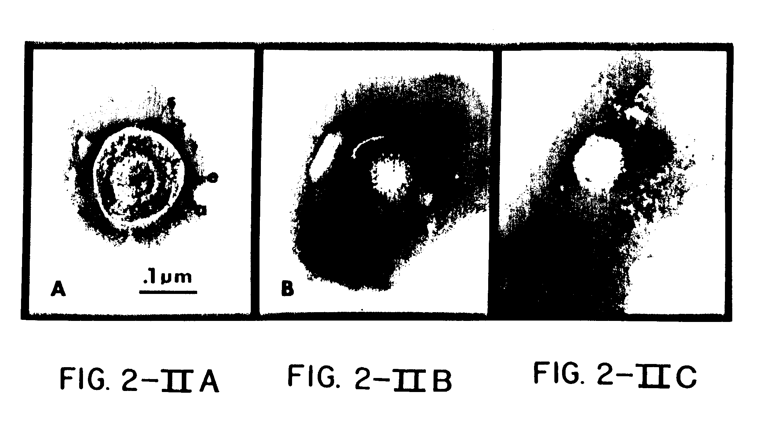

Electron microscope examination (Fig. 2-IIa) shows that these cells were infected by an enveloped DNA virus, 200 nm in diameter. These large cells were also the only ones in culture expressing viral antigens, as measured by fixed cell indirected immunofluorescence assays (IFA). All three virus-positive patients were homosexual males (2 white and 1 black, between the ages of 35 and 44), who were seropositive for HTLV-III with AIDS-pneumoncytsis pneumonia, with Kaposi's sarcoma, and with undifferentiated B-cell lymphoma.

The presence of the unique large, refractile cells suggested the need for further examination of patients demonstrating morphologically similar cells in fresh or cultured tissues.

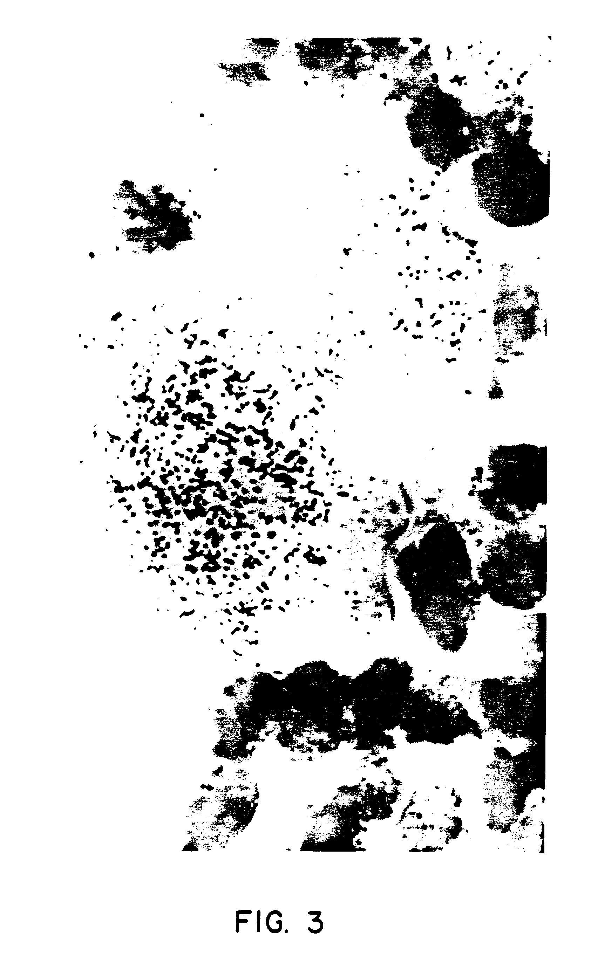

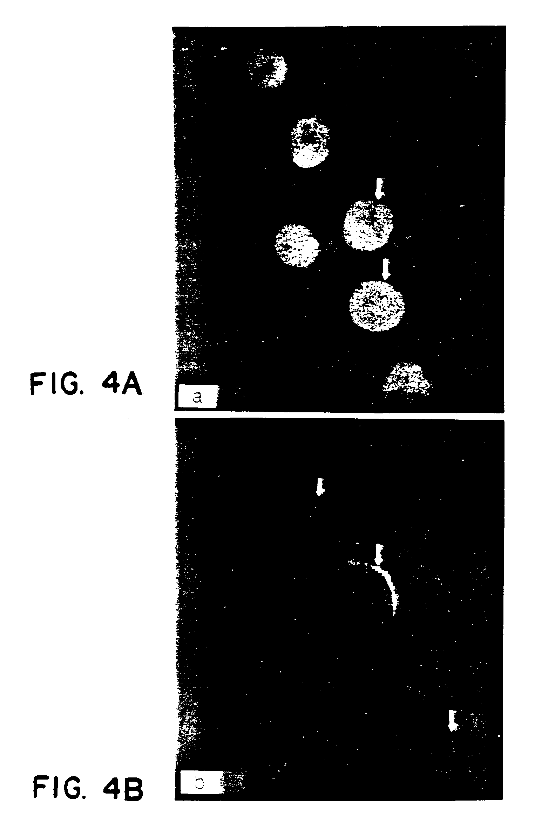

HBLV from all six patients could be transmitted to freshly isolated human leukocytes from umbilical cord blood, adult peripheral blood, bone marrow, and spleen (previously stimulated with PHA-P (phytohemagglutinin-purified) as described in the Specific Disclosure). After in vitro infection in the large refractile cells, noted in primary cultures, appeared within 2-4 days post infection (Fig. 1b). This cell eventually became the predominant cell in the culture surviving for an additional 8-12 days. During this time other cells in culture rapidly died. As in primary cell cultures, these large cells expressed viral nucleic acids as shown by in situ hybridization (Fig. 3), and viral antigens as detected by IFA (Figs. 4a and b). Virus production was confirmed by electron microscopy (Fig. 2b). HBLV-infected cells were typed for surface markers defined by specific monoclonal antibodies, and were found to express antigens recognized by B-cell specific Leu-12, Leu-16, B1, and B4 monoclonal antibodies. These cells lacked T-cell markers as measured by OKT-3, OKT-4, and OKT-8 monoclonal antibodies.

Molecular probes specific for HSV-1, CMV, and EBV were used for comparisons with HBLV. While each individual viral probe specifically hybridized to its homologous nucleic acids, HBLV was clearly distinct from these transforming human DNA viruses. Furthermore, the size of the HBLV genome was shown to contain a minimum complexity of 110 Kb-pair as determined by analysis of sucrose gradient purified viral DNA. This genome size, as well as other features, also distinguishes HBLV from DNA viruses of the adenovirus, polymavirus, papovavirus, and papillomavirus groups.

## DESCRIPTION OF THE FIGURES

Figure 1 is the peripheral blood leukocytes from a patient with AIDS-associated lymphoma (a) and HBLV-infected umbilical cord blood leukocytes in cell culture (b).

Figure 2 is an electron micrograph of a cultured cell producing HLBV: I is peripheral mononuclear blood cells, and II is PHA-P stimulated umbilical cord blood mononuclear cells.

Figure 3 is in situ hybridization of HBLV-infected human cord blood cells.

Figure 4 is an immunofluorescent analysis of HLBV-infected cells.

Figure 5 is a southern blot analysis of HBLV genomic DNA.

Figures 6-12 are gel electrophoresis patterns of proteins recongized by human sera against HBLV.

## STATEMENT OF DEPOSIT

A molecular clone called pZVH14 has been deposited in the American Type Culture Collection in Rockville, Maryland under ATCC No. 40247, and at issuance of this application into a patent, will be maintained for a term of thirty (30) years from the date of deposit or five (5) years from the date of the last request for such deposit or for the effective life of this patent, whichever is longest. The deposit will be replaced if the culture mutates or becomes nonviable during the term of the deposit.

## SPECIFIC DISCLOSURE

Despite morphological and other properties similar to some of the Herpesviruses, Human B Lymphotropic Virus (HBLV) appears to be a new human DNA virus. It is distinguishable from other viruses by biological properties and by lack of immunological and genomic homology. HBLV is highly lytic in vitro, as are CMV, HSV (herpes simplex virus), HVS, and HVA, but has a narrower host range than these viruses or EBV, being limited to a subset of B- cells. It is possible that HBLV could indirectly cause abnormalities in B-cells leading to malginancy in vivo.

Even though in the preliminary studies HBLV was associated in five (5) instances with HTLV-III/LAV seropositive donors, other evidence indicates that it is not exclusively an AIDS-associated agent. Not only did all HTLV-III seropositive patients have complicating lympho-proliferative disorders, but HBLV was also isolated from a HTLV-III seronegative ALL patient. Furthermore, preliminary seroepidemiological analysis has shown a reactivity clearly dissociated from HTLV-III individuals.

4

Serological comparisons demonstrate the uniqueness of HBLV. Immunofluorescence assay was developed following techniques originally described for Herpesviruses, and was used to analyse patients and healthy control sera, and to monitor infected cells. Sera from all six HBLV positive patients demonstrated an IgG antibody titer to viral capsid antigens (>1:20). In contrast, only 4 of the more than 200 sera from randomly selected healthy donors were positive. The pattern of immunofluorescent staining in fixed, infected cells varied from punctate nuclear staining to diffuse staining of the entire cell (Fig. 4a). In live cells, the staining was confined to the cell membrane either as a partial ring or in a capped form (Fig. 4b). Uninfected cord blood mononuclear cells were negative when tested with sera from the 6 HBLV positive patients. Sera from these positive patients also contained antibody to EBV and CMV. A careful comparison of the titers of antibody to EBV, CMV, and HBLV yielded a distinct titer for HBLV as compared to that for EBV and CMV. Furthermore, the reactivity to EBV was completely removed by adsorption with disrupted, EBV-infected cells or with purified EBV, without significantly affecting the antibody titer to HBLV.

Sucrose gradient purification of HBLV. Heparinized peripheral blood leukocytes or human umbilical cord blood mononuclear cells are banded in Ficoll-Hypaque and established in cell culture at 36°C following PHA-P (5μg/ml) stimulation for 48 hours. The cells are then grown in RPMI-1640 supplemented with 10% fetal bovine serum (heat inactivated, 56°C for 30 min.) and 5μg/ml hydrocortisone. Frozen supernatants obtained from the infected cells are thawed, collected in 250 ml tubes and spun at 3500 rpm in a Sorall GSA rotor at 5°C for 10 min. The clarified supernatants are transferred to SW28 tubes and spun and pelleted at 17,000 rpm for 90 min. at 5°C. Pellets obtained are resuspended in 10 mM Tris-HCl pH 7.4, 10 mM NaCl, 1 mM EDTA (TNE) to a volume of 300 microliters and layered onto a 15-60% sucrose gradient and spun in an SW41 rotor (Beckman) at 20,000 rpm for 30 min at 5°C. Fractions of 1 ml are collected from the top of the gradient. Each fraction is diluted to 10 ml and spun and pelleted in an SW41 rotor at 17,000 rpm for 90 min. Pellets are resuspended in 300 microliters of TNE and aliquots assayed (by ELISA and Western Blot) for the presence of virus and for virus infectivity. Human B Lymphotropic Virus is easily detected in fractions 4-9 with a peak in fractions 5-7 by both assays. Extraction of nucleic acids from each fraction shows the presence of double stranded DNA in fractions 5-9 with a peak in fraction 7. Virus is also detected by electron microscopy in the SW41 gradient pellet as well.

Virus purified from fresh supernatants according to the above procedure is used for detailed electron microscopy.

Aliquots of the sucrose gradient fractions can be assayed for the presence of HBLV by DNA dot blot analysis using the pZVH14 9 Kb insert as a probe. The pZVH14 molecular clone may be obtained from the American Type Culture Collection under Accession No. 40247.

The immunofluorescence assay and Western Blot assay are the preferred assays for detecting HBLV infection and HBLV antibodies in a variety of hematopoietic malignancies, including B-cell lymphomas of both AIDS and non-AIDS origin. The presence of HBLV antibodies is elevated in the following disease groups, but the invention is not intended to be limited to these specific diseases:

Burkitts lymphomas;

Hodgkin's disease;

A newly described infectious disease syndrome similar to that seen in Lake Tahoe characterized as an "acute mononucleosis-like syndrome" in adults; and

ALL as diagnosed in children of Caribbean and African origin.

HBLV Virus Propagation. Infection of human umbilical cord blood or peripheral blood mononuclear cells is conducted by cell-free transmission as follows:

(1) Fresh blood samples are diluted 1:1 with RPMI-1640 and spun (and banded) on a Ficoll gradient.

(2) The banded mononuclear cells are washed and put into culture in the presence of PHA-P (5μg/ml) and HC (5μg/ml) in 20% FCS and RPMI-1640.

(3) After 24 hours, polybrene (2μg/ml) is added to the culture and after 48 hours, the cells are pelleted.

(4) A 1 ml aliquot of freshly harvested or frozen infected culture supernatant is added to the pellet and incubated at 37°C for 1 hour, with frequent agitation.

(5) Fresh medium [10% FCS and HC (5 μg/ml) in RPMI-1640] is then added to the suspension, cultured, and incubated at 36°C.

(6) Within 2-10 days post infection, the characteristic enlarged refractile cells become visible. Supernatant is harvested at the peak of infection as measured by immunofluorescence and by visual observation of the culture for further transmission.

Cells infected by HBLV were also used to directly compare immunological cross-reactivities with other human and nonhuman primate Herpesviruses using specific monoclonal antibodies, hyperimmune sera, or sera from antibody positive control donors. As summarized in Table 3, all monoclonal antibodies to EBV, CMV, HSV, and hyperimmune sera to Rhesus CMV and African Green CMV, did not react with HBLV-infected cells. Furthermore, sera from several old world and new world primates many of which had antibodies to nonhuman primate Herpesviruses (including EBV-like viruses and CMV) did not show any cross-reactivity with HBLV-infected cells (Table 2).

Immunofluorescent Analysis of HBLV-Infected Cells. A modification of the indirect immunofluorescence assay developed by Henle, et al. was used for the detection of antibody to HBLV capsid antigens. For this assay, HBLV-infected or uninfected human cord blood mononuclear cells were washed 3 times for 10 minutes with PBS, resuspended in PBS, deposited on teflon coated slide, air dried, and fixed in cold acetone for 10 minutes. Patient's sera (heat inactivated at 56°C for 30 min. and clarified by centrifugation) were added to the fixed cells, incubated in a humidity chamber at 37°C for 40 min., and washed with PBS, air dried, and stained with affinity purified FITC conjugated anti-human IgG (H and L) for 40 min. The cells were again washed as above, air dried, and mounted with IF mounting solution. Larges cells with granular immunofluorescent staining, and cytoplasmic infected cells with HBLV as shown in Figure 1b were assayed 5 days post injection are shown in Figure 4. Small cells in the background did not react with patient serum.

As is shown in Fig. 4b, detection of viral membrane antigen HBLV infected as well as uninfected live cells (non-fixed) were washed 3 times in serum-free medium, treated with patient's serum for 30 minutes at 4°C. The cells were again washed, treated with affinity purified FITC anti-human IgG for another 30 minutes, washed in medium again and examined for membrane fluorescence.

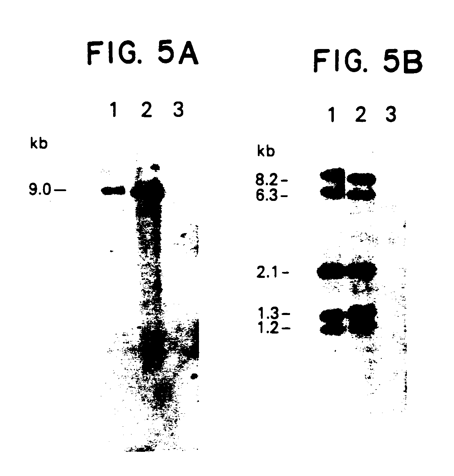

Southern Blot Analysis of HBLV Genomic DNA. Supernatant fluid from HBLV infected umbilical cord blood cells is layered onto 20% glycerol cushions and pelleted by centrifuging at 25,000 rpm for 3 hr. in a Beckman SW41 rotor at 4°C. The pellets are suspended up in TNE buffer (10 mM, Tris-HCl, pH 9, 100 mM NaCl:1 mM EDTA), and extracted with PCI9 (Phenl:Choloroform:Isoamyl alcohol; 50 mM Tris-HCl, pH 9:: 100:100:1:10 :: v:v:v:v) followed Choloroform:isoamyl alcohol (24:1::v:v). Enriched viral DNA is precipitated by adding 2 volumes of 95% ethanol. DNA is digested with HindIII and cloned into the Bluescrib vector (commercially available from Vector Cloning Systems, CA). Several clones were obtained after screening with labeled, enriched DNA were examined for specificity of hybridization to infected human umbilical cord blood cell DNA and by in situ hybridization to infected cells. Specific hybridization of HBLV clone pZVH14 to DNA from pelleted virus digested with HindIII (Fig. 5, panel a) and EcoR1 (Fig. 1, panel b). Extracellular virus is shown in lane 1, virus infected human umbilical cord blood cells in lane 2, and negative control DNA isolated from the skin of an AIDS patient in lane 3. Clone pZVH14 scored positive in these assays and did not hybridize to uninfected controls. The infected cell DNA shown in lane 2 is isolated after several rounds of cell free virus transmission in human umbilical cord blood cells.

EXAMPLES

Example 1. Fresh tissue sections from 3 patients were found to contain a low number of HBLV-infecteed cells. One patient, a 40 year old Hispanic with a history of IV drug use, was seropositive for both HTLV-I and HTLV-III, and was diagnosed with AIDS-pneumoncystic pneumonia with associated dermatophathic lymphadenopathy. Another was a 61 year old white male who received multiple blood transfusions in conjunction with open heart surgery 4 years prior to death. This patient was seropositive for HTLV-III and was diagnosed with immunoblastic lymphadenopathy with some skin involvement. A third patient was a 16 year old black male diagnosed with acute lymphocytic leukemia of the T-cell type. Unlike the others, this patient was seronegative for HTLV-III. Primary peripheral blood mononuclear cell cultures from these patients also contained a small number of the unique cell which, upon close examination were also found to be infected by HBLV.

Example 2. A direct comparison of molecularly cloned sequences of the HBLV genome (Salahuddin, et al., "Isolation of a New Human B Lymphotropic Virus (HBLV) from Patients with AIDS-Associated and Other Malignancies", Science, Volume 234, pp. 596-601) with the genomes of other Herpesviruses was also conducted. Several DNA clones obtained from nucleic acids extracted from purified virus were examined for specificity and for comparison with other DNA viruses. One HBLV clone, designated pZVH14, which contained a 9.0 Kb HindIII fragment, was used in these studies. Southern Blot analysis (Fig. 5) showed the presence of viral specific DNA in HindIII and EcoRI digests of DNA from both purified virus and HBLV-infected human cord blood cells. In situ hybridization experiments with the same probe also confirmed that these sequences were confined to the infected cells (Fig. 3).

Example 3. Monoclonal antibodies and hyperimmune sera prepared against human and simian Herpesviruses were tested for reactivity with HBLV infected cells by indirect immunofluorescence procedures as described.

Monoclonal antibodies to EBV and HCMB were used at 1:40 dilution; HSV-I and II, VZV and HVS at a 1:10 dilution normal ascites fluid was used at 1:5 and 1:10 dilutions. Hyperimmune sera to African green and Rhesus monkey CMV were heat inactivated (50°C 30 min.) and clarified at 10,000 rpm and were used at 1:10 dilutions. In addition to the sera shown, human sera containing antibodies to EBV, CMV, HSV-I and II, and VZV also did not react with HLV infected cells. African green monkey and Rhesus sera containing antibody to CMV were also negative when tested with HBLV. Monoclonal antibodies to EBV, and HCMV, and ascites fluid from normal mouse were gifts from Dr. Gary Pearson, School of Medicine, Georgetown University, Washington, D.C. Monoclonal antibodies to VZV and HVS were obtained from Dr. Nancy Chang, Baylor College of Medicine, Houston, Texas, and Dr. John Dahlberg, NCI, Bethesda, Maryland. HSV-I and II monoclonal antibodies were purchased from Dupont, Boston, MA. Hyperimmune serum to purified African green and Rhesus CMV were previously prepared in rabbits by Dr. Ablashi.

Abbreviations used: HBLV, Human B Lymphotropic Virus; EBV, Epstein-Barr Virus; HCMV, Human Cytomegalovirus; HSV, Herpes Simplex Virus; VZV, Varicella-Zoster Virus; HVS, Herpes Virus Saimiri; VCA, .op viral capsid antigen; EA, early antigen; MA, membrane antigen.

HBLV infected cord blood mononuclear cells were stained with an HBLV negative serum resulting in a considerable number of large cells with no immunofluorescence. The results are tabulated in Table 3.

Example 4. Serum from old world and new world primates were tested for antibody to HBLV by indirect immunofluorescence as described.

Some sera from the old world primates were gifts from Dr. P. Kanki, Harvard School of Public Health, Boston, MA. All sera were heat inactivated at 56°C for 30 min., clarified by centrifugation before use. HBLV-infected cord blood leukocytes, P3HR-1 (an established cell line expressing EBV-VCA), and Owl monkey kidney cells infected by HSV-strain II were used for comparisons. When infected cells showed cytopathic effects, the cells were fixed in acetone and used for the IFA.

Three owl monkeys and one cottontop marmoset were previously innoculated with HVS. Sera from these animals possessed antibody to HVS late antigen which cross-reacted with Herpesvirus ateles. The results are tabulated in Table 2.

Example 5. In situ hybridization of HBLV-infected human cord blood cells. Experiments were performed utilizing $^{35}$S-labeled RNA probes as described in the Specific Disclosure. Clone pZVH14 of the HBLV genome was used as template for radiolabeled RNA using T7 RNA polymerase, $^{35}$S-labeled dGTP, and unlabeled ribotriphosphates. Less than one grain per cell was observed in uninfected negative control cultures. Large refractile cells characteristic of the infected cultures were heavily labeled, indicating the expression of abundant viral messages.

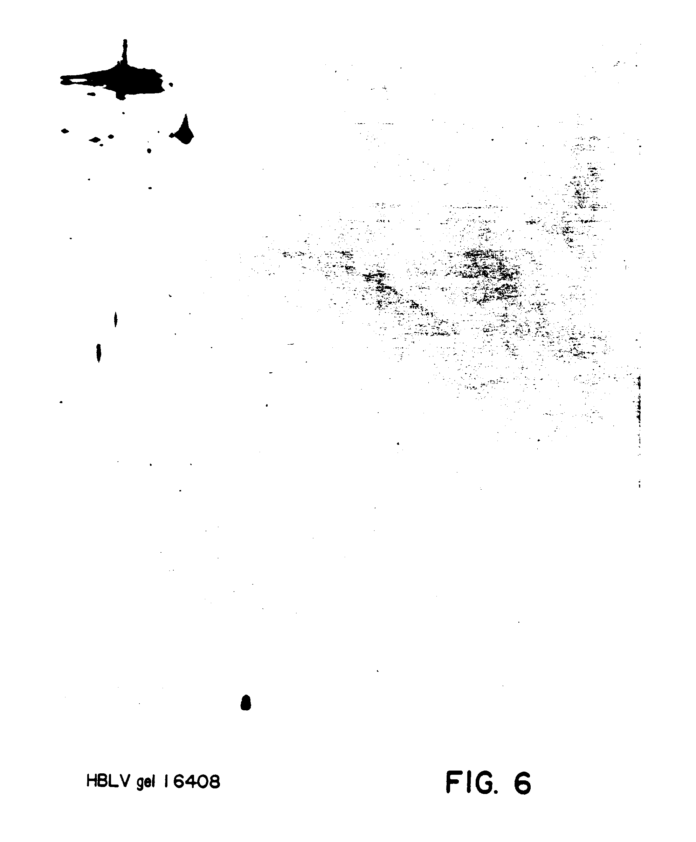

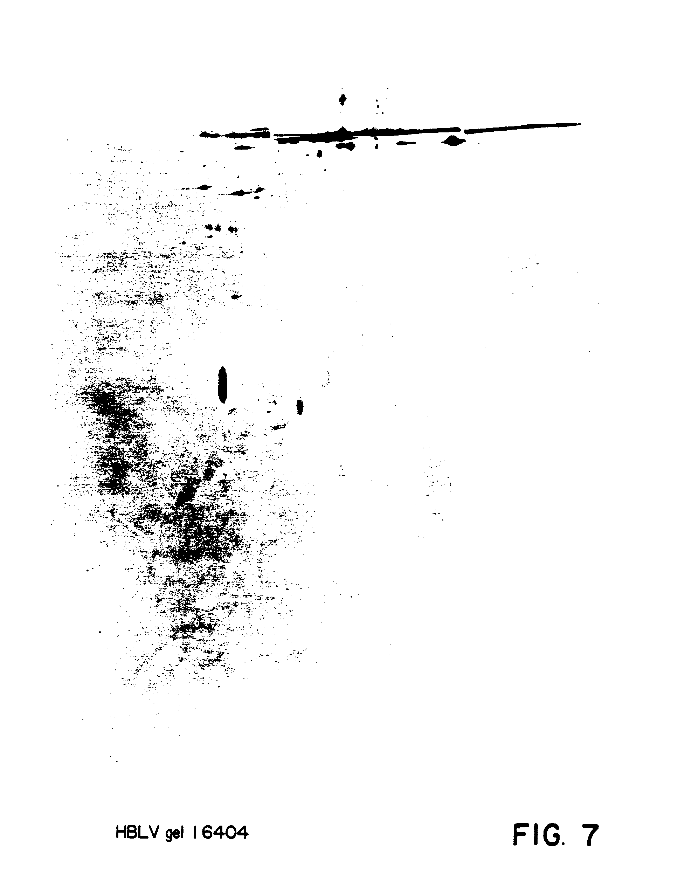

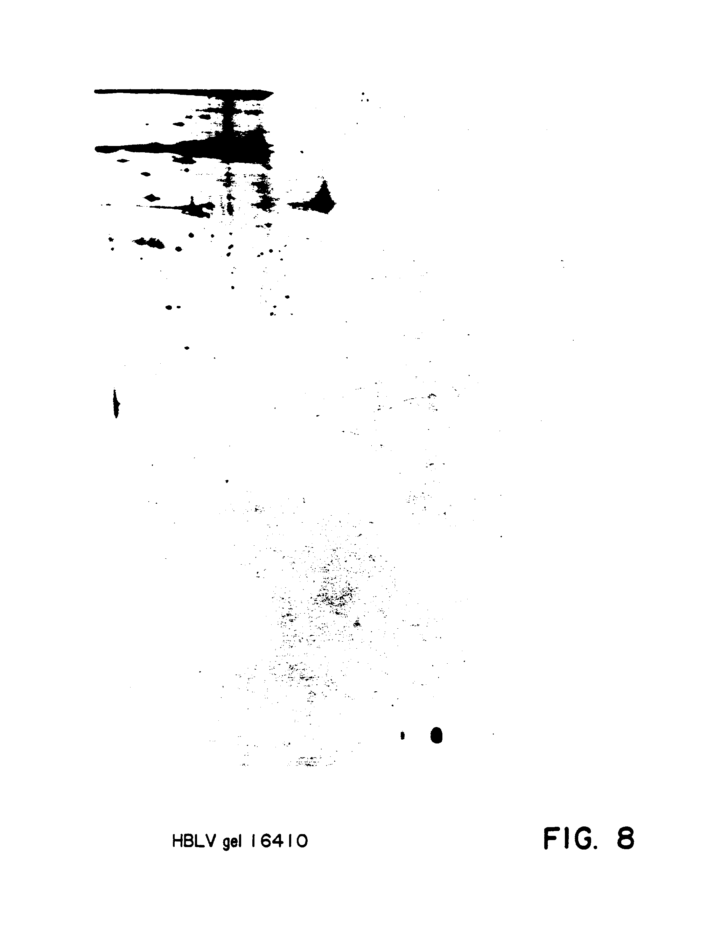

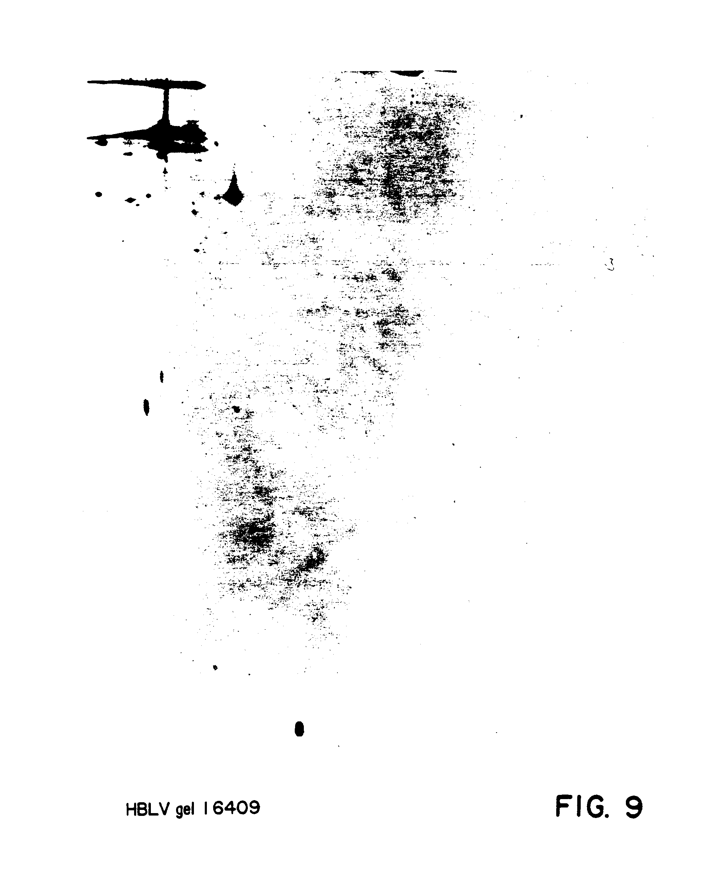

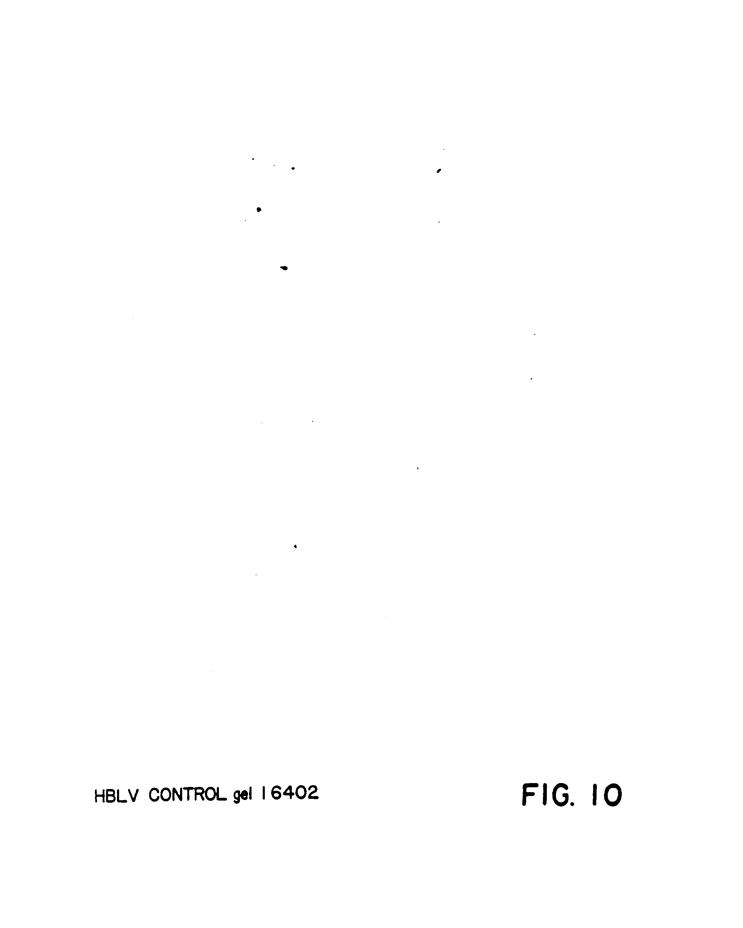

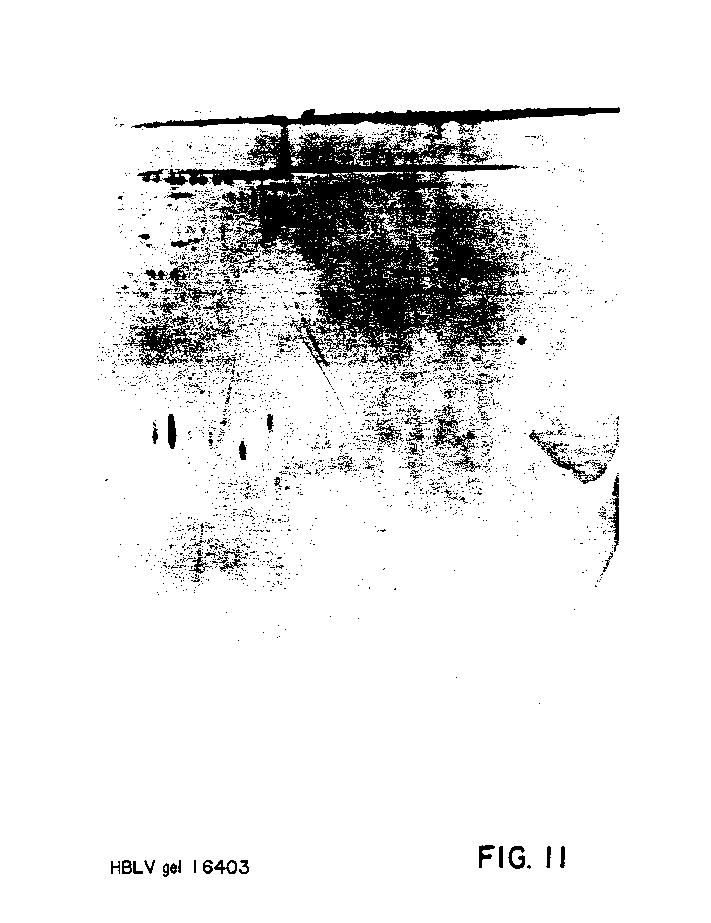

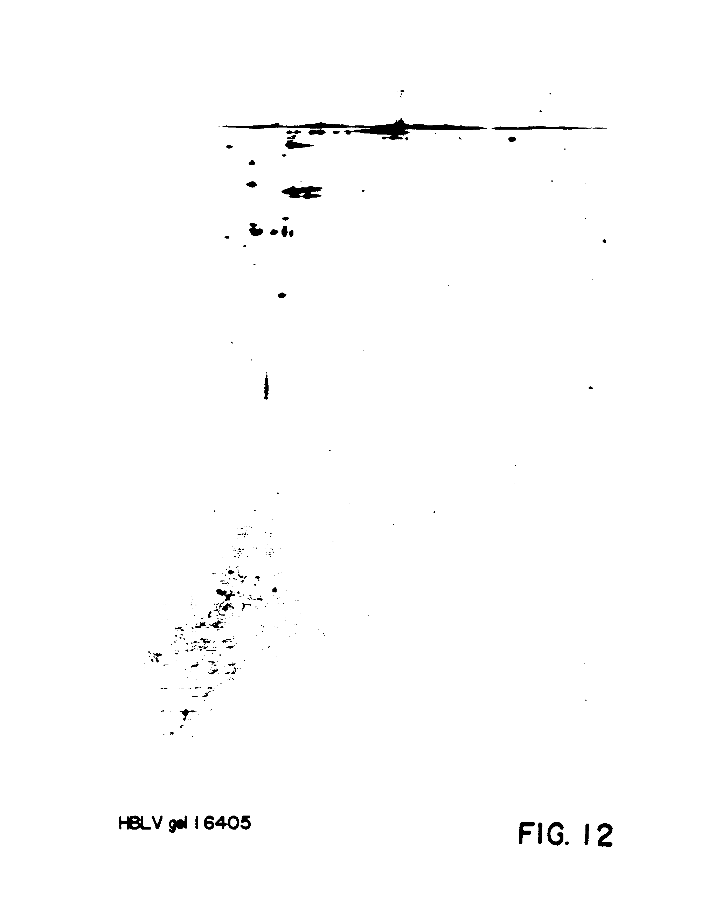

Example 6. Two dimensional gel electrophoresis patterns of proteins recognized by human sera against human B cell lymphotropic virus (HBLV) are shown in Figures 6-12. Human umbilical cord blood lymphocytes were infected with HBLV and then labeled by incubation with $^{35}$S-methionine for periods of either 3 hours or 24 hours. H9 cells were used as negative controls. The labeled cells were lysed and the proteins immunoprecipitated according to established procedures (Protein Data Bases, Inc., New York). Spots seen on the gels of the lysates from infected cells but not seen on the control gels represents candidate virus proteins arrayed in unique virus specific patterns. These patterns serve as a fingerprint which can specifically identify HBLV. Included are Gels Nos. 16408 (Figure 6), 16404 (Figure 7), 16410 (Figure 8), 16409 (Figure 9), 16402 (Figure 10), 16403 (Figure 11), and 16405 (Figure 12).

## TABLE 2

### Cross-Reactivity of Nonhuman Primate Sera

#### Virus Used to Infected Target Cells

| Serum Sources | HBLV No. Positive/ No. Tested (Percent Positive) | | EBV No. Positive (VCA)/No. Tested (Percent Positive) | | HSV No. Positive/ No. Tested (Percent Positive) | |
|---|---|---|---|---|---|---|
| **Old World Primates** | | | | | | |
| Chimpanzee | 0/5 | (0) | 5/5 | (100%) | 0/4 | (0) |
| Gorilla | 0/3 | (0) | 2/3 | (66.6%) | 0/3 | (0) |
| Orangutan | 0/2 | (0) | 1/2 | (50%) | 0/2 | (0) |
| Baboons | 0/3 | (0) | 3/3 | (100%) | 0/3 | (0) |
| Stumptail | 0/2 | (0) | 1/2 | (50%) | 0/2 | (0) |
| Rhesus | 0/9 | (0) | 6/9 | (66.6%) | 0/7 | (0) |
| African Green | 0/10 | (0) | 6/10 | (60%) | 0/10 | (0) |
| **New World Primates** | | | | | | |
| Squirrel Monkeys | 0/10 | (0) | 0/10 | (0%) | 8/10 | (80) |
| Owl Monkeys | 0/6 | (0) | 0/6 | (0%) | 3/6 | (50) |
| Marmosets (common) | 0/6 | (0) | 0/6 | (0) | 0/6 | (0) |
| Marmosets (cottontop) | 0/3 | (0) | 0/3 | (0) | 1/3 | (33.3) |

EP 0 318 498 B1

TABLE 3

Immunological Cross Reactivities of HBLV to Other Human and Nonhuman Primates Herpesviruses Antibody Viruses Used to Infect Target Cells

| Source | HBLV | EBV | HCMV | HSV-I and II | VZV | HVS | Af. Gr. CMV | Rhesus CMV |
|---|---|---|---|---|---|---|---|---|
| EBV Monoclonal Antibody (VCA,EA,MA) | - | + | - | - | - | - | - | - |
| HCMV Monoclonal Antibody (VCA and EA) | - | - | + | - | - | - | - | - |
| HSV I and II Monoclonal Antibody (early and late antigens) | - | - | - | + | - | - | - | - |
| VZV Monoclonal Antibody (late antigens) | - | - | - | - | + | - | - | - |
| HVS Monoclonal Antibody (late antigens) | - | - | - | - | - | + | - | - |
| Af. Green Monkey CMV (hyperimmune serum) | - | - | - | - | - | - | + | - |
| Rhesus Monkey CMV (hyperimmune serum) | - | - | - | - | - | - | - | + |

## Claims

1. A method of obtaining HBLV (Human B Lymphotropic Virus) virus particles by the following steps:
   1. growing a co-culture of infected patient blood with uninfected suitable target cells;

9

2. isolating the infected cells from the culture cell cloning;

3. multiplying infected cells by serial transmission;

4. isolating virus from infected cell culture; and

5. concentrating the virus.

2. The method of Claim 1 wherein the target cells are uninfected B lymphocytes from cord blood.

3. A method of producing HBLV virus proteins wherein, in addition to the steps of Claim 1, the virus proteins are isolated by gel electrophoresis from the virus-infected cell culture of step 4.

**Patentansprüche**

1. Verfahren zur Gewinnung von HBLV(menschlicher B-lymphotroper Virus)-Viruspartikeln, mit den folgenden Verfahrensschritten:

1. Züchten einer Co-Kultur des Blutes infizierter Patienten mit nichtinfizierten geeigneten Targetzellen;

2. Isolieren der infizierten Zellen von den Zellkulturklonierung

3. Vermehren der infizierten Zellen durch serielle Übertragung;

4. Isolieren des Virus von der infizierten Zellkultur; und

5. Konzentrieren des Virus.

2. Verfahren nach Anspruch 1, wobei die Targetzellen von nichtinfizierten B-Lymphozyten aus dem Blut der Nabelschnur gebildet sind.

3. Verfahren zur Herstellung von HBLV-Virusproteinen, bei dem zusätzlich zu den Verfahrensschritten von Anspruch 1 die Virusproteine durch Gelelektrophorese von der virusinfizierten Zellkultur des Verfahrensschrittes 4 isoliert werden.

**Revendications**

1. Méthode d'obtention de particules de virus HBLV (virus humain B lymphotrope) selon les étapes suivantes :

1.Croissance d'une co-culture de sang de patient infecté avec des cellules cibles appropriées non infectées;

2.Isolement des cellules infectées à partir du clonage de la culture cellulaire;

3.Multiplication des cellules infectées par transsission successive;

4.Isolement du virus à partir de la culture cellulaire infectée; et

5. Concentration du virus.

2. Méthode selon la revendication 1 dans laquelle les cellules cibles sont des lymphocytes B de sang cordonnal non infecté.

3. Méthode de production de protéines de virus HBLV dans laquelle, outre les étapes de la revendication 1, les protéines virales sont isolées par électrophorèse sur gel à partir de la culture cellulaire infectée par le virus de l'étape 4.

FIG. 1A

FIG. IB

FIG. 2-IC   FIG. 2-IB   FIG. 2-IA

FIG. 2-IF   FIG. 2-IE   FIG. 2-ID

FIG. 2—IIA          FIG. 2—IIB          FIG. 2—IIC

FIG. 3

FIG. 4A

FIG. 4B

# FIG. 5A

1  2  3

kb

9.0 —

# FIG. 5B

1  2  3

kb

8.2 –
6.3 –

2.1 –

1.3 –
1.2 –

HBLV gel 16408

FIG. 6

HBLV gel I 6404

FIG. 7

HBLV gel 16410

FIG. 8

HBLV gel I 6409

FIG. 9

HBLV CONTROL gel 16402

FIG. 10

HBLV gel I 6403

FIG. II

EP 0 318 498 B1

HBLV gel I 6405

FIG. 12